# EUROPÄISCHE PATENTSCHRIFT

(11) **EP 3 281 601 B1**
(45) Veröffentlichungstag und Bekanntmachung des Hinweises auf die Patenterteilung: **07.11.2018**
(21) Anmeldenummer: 16183329.8
(22) Anmeldetag: 09.08.2016
(51) Int. Cl.: A61B 46/10, A61B 46/00, A61F 7/00

(54) **KUNSTSTOFFAUSKLEIDUNG UND VERFAHREN ZUR HERSTELLUNG**
PLASTIC LINER AND METHOD FOR PRODUCING SAME
PROTECTION EN PLASTIQUE ET PROCEDE DE FABRICATION

(43) Veröffentlichungstag der Anmeldung: 14.02.2018
(73) Patentinhaber: Sensoproducts GmbH, 1050 Wien (AT)
(72) Erfinder: Mandl, Rene, 1050 Wien (AT)
(74) Vertreter: Puchberger & Partner Patentanwälte

(56) Entgegenhaltungen:
- EP-A1- 2 524 665
- DE-T2- 69 830 725
- US-A- 5 457 962

## Beschreibung

Die Erfindung betrifft eine Kunststoffauskleidung für eine beheizbare Schale, umfassend einen der Form der Schale anpassbaren Schalenbereich und einen an den Schalenbereich anschließenden Schürzenbereich zur Abdeckung eines Unterbaus der Schale. Die Erfindung betrifft weiters ein Verfahren zur Herstellung einer derartigen Kunststoffauskleidung.

Derartige Kunststoffauskleidungen sind aus dem Stand der Technik zum Einsatz bei chirurgischen Operationen bekannt. Statt die Schale selbst nach jeder Operation zu reinigen und zu desinfizieren, wird der Schalenbereich der sterilen Kunststoffauskleidung in die Schale eingebracht, der Schürzenbereich über den Unterbau der Schale geklappt, und die benötigte Flüssigkeit, beispielsweise eine sterile Kochsalzlösung, in den Schalenbereich gefüllt. Die Kunststoffauskleidung kann nach der Verwendung entsorgt werden.

Bei Operationen verwendete Schalen sind in der Regel aus Metall und weisen einen beheizten Boden auf, um die Flüssigkeit auf Körpertemperatur vorzuwärmen. Dabei stellt sich das Problem, dass die Kunststoffauskleidung in Kontakt mit dem beheizten Boden der Metallschale tritt und dadurch beschädigt wird. Darüber hinaus hat sich in der Praxis auch gezeigt, dass die Kunststoffauskleidung im Schalenbereich erhöhten mechanischen Belastungen, beispielsweise durch chirurgische Instrumente, standhalten muss.

In der EP 2 524 665 A1 wird zur Behebung dieser Probleme eine gattungsgemäße Kunststoffauskleidung für eine beheizte Metallschale vorgeschlagen, bei der die Kunststoffauskleidung im Schalenbereich mehrlagig ist und überdies einen Aluminium-Zuschnitt als Wärmeschutzschicht aufweist. Durch die Mehrlagigkeit im Schalenbereich wird gewährleistet, dass selbst bei einer mechanischen Beschädigung der Innenschicht immer noch die Dichtheit der Auskleidung gegeben ist. Der Aluminium-Zuschnitt dient als Wärmeschutzschicht, um den Kunststoff gegen Temperaturspitzen zu schützen.

Auch diese Kunststoffauskleidung leidet jedoch unter einigen Nachteilen. So führt die Mehrlagigkeit im Schalenbereich zwangsläufig dazu, dass die Übertragung der thermischen Energie von der Metallschale an die zu erwärmende Flüssigkeit ungleichmäßig erfolgt, da sich zwischen den einzelnen Lagen der Auskleidung isolierende Hohlräume oder Falten bilden können. Außerdem ist der Aluminium-Zuschnitt nicht transparent, sodass der Benutzer weder den Zustand der Schale selbst überprüfen kann, noch, ob die Auskleidung im Schalenbereich unmittelbar mit der beheizten Schale in Kontakt steht. Es können sich Falten oder Fremdkörper zwischen dem Aluminium-Zuschnitt und der Metallschale befinden, die von außen nicht erkennbar sind. Außerdem führt die sehr hohe Wärmeleitfähigkeit des Aluminium-Zuschnitts zwangsläufig dazu, dass Temperaturspitzen besonders gut an die innere Lage der Kunststoffauskleidung weitergeleitet werden, sodass der Aluminium-Zuschnitt seine eigentliche Aufgabe als Wärmeschutzschicht nur ungenügend erfüllt.
Herkömmliche Kunststoffauskleidungen sind unter anderem aus der EP 2 524 665 A1, der US 5,457,962 A und der DE 698 30 725 T2 bekannt. Die objektive Aufgabe der Erfindung besteht somit darin, diese und andere Nachteile herkömmlicher Kunststoffauskleidungen zu beheben. Dies wird durch eine Kunststoffauskleidung gemäß Anspruch 1 sowie ein Verfahren zur Herstellung einer erfindungsgemäßen Kunststoffauskleidung gemäß Anspruch 10 erreicht. Die erfindungsgemäße Kunststoffauskleidung umfasst einen Schürzenbereich, der Polyethylen umfasst oder als Polyethylen-Folie ausgebildet ist. Der an den Schürzenbereich anschließende Schalenbereich umfasst einen Seitenwandbereich zur Abdeckung der Seitenwand der Schale, sowie einen Bodenbereich zur Abdeckung des Bodens der Schale. Dabei beinhaltet der Seitenwandbereich zumindest Polyamid und Polyethylen, und ist insbesondere als Folie aus einem Polyamid-Polyethylen Verbund ausgebildet.

Erfindungsgemäß beinhaltet der Bodenbereich zumindest ein temperaturbeständiges Fluorpolymer, insbesondere Tetrafluorethylen-Hexafluorpropylen-Copolymer (FEP) oder Polytetrafluorethylen (PTFE).

Der Seitenwandbereich und der Bodenbereich sind als flexible, transparente Folien ausgebildet, um eine Anpassung der Kunststoffauskleidung an die Form der Schale zu ermöglichen.
Indem der Bodenbereich ein Fluorpolymer, insbesondere Tetrafluorethylen-Hexafluorpropylen-Copolymer (FEP) oder Polytetrafluorethylen (PTFE) umfasst, wird eine hohe Temperaturbeständigkeit der Kunststoffauskleidung im Bodenbereich gewährleistet.
Erfindungsgemäß kann der Bodenbereich als vorzugsweise thermischer Verbund aus einer FEP- oder PTFE-Folie mit einem anderen Kunststoff, beispielsweise Polyethylen, ausgebildet sein. In diesem Fall kann erfindungsgemäß vorgesehen sein, dass die FEP-oder PTFE-Folie an jener Seite des Bodenbereichs vorgesehen ist, die bei Benutzung in Kontakt mit der beheizten Schale steht.
Erfindungsgemäß kann vorgesehen sein, dass der Seitenwandbereich, der Bodenbereich und der Schürzenbereich der Kunststoffauskleidung jeweils einlagig sind. Dadurch wird verhindert, dass sich zwischen einzelnen Lagen Hohlräume oder Fremdkörper bilden können, die eine optimale Wärmeübertragung an die zu erwärmende Flüssigkeit verhindern könnten.
Erfindungsgemäß kann vorgesehen sein, dass der Schürzenbereich eine Dicke im Bereich von 40µm bis 80 µm, vorzugsweise 60 µm aufweist.
Erfindungsgemäß kann vorgesehen sein, dass der Seitenwandbereich eine Dicke im Bereich von 120 µm bis 180 µm, vorzugsweise 150 µm, aufweist.
Erfindungsgemäß kann vorgesehen sein, dass der Bodenbereich eine Dicke im Bereich von 200 µm bis 300 µm, vorzugsweise 260 µm, aufweist.

Indem der Bodenbereich dicker als der Seitenwandbereich, und der Seitenwandbereich dicker als der Schürzenbereich ausgeführt wird, wird gewährleistet, dass der Seitenwandbereich und insbesondere der Bodenbereich eine ausreichende Stabilität gegenüber mechanischen Beanspruchungen durch eingelegte Operationsinstrumente bietet, gleichzeitig jedoch die gesamte Kunststoffauskleidung leicht und kompakt bleibt, um in einer üblichen sterilen Verpackung gelagert zu werden.

Der Bodenbereich kann insbesondere kreisförmig mit einem Durchmesser von 20 cm bis 30 cm, vorzugsweise etwa 26 cm ausgeführt, sein. Der an den Bodenbereich anschließende Seitenwandbereich kann eine Höhe bzw. Erstreckung von 5 cm bis 15 cm, vorzugsweise etwa 7 cm aufweisen, und sich zylindrisch oder konisch nach außen erweitern. Schließlich kann der Schürzenbereich eine wesentlich größere Erstreckung, beispielsweise 50 cm bis 100 cm, vorzugsweise etwa 75 cm, aufweisen und sich ebenfalls vorzugsweise konisch erweitern, um das Umklappen über den Rand der Schale zu erleichtern.

Erfindungsgemäß kann vorgesehen sein, dass der Schalenbereich an den Schürzenbereich angeformt, insbesondere über eine umlaufende thermische Schweißnaht mit dem Schürzenbereich verbunden ist.

Erfindungsgemäß kann vorgesehen sein, dass der Seitenwandbereich einen Verbund aus Polyamid mit einer beidseitigen Versiegelung aus Polyethylen umfasst. Der Setienwandbereich kann insbesondere aus dem Material NICE XX8 des Unternehmens WIPAK hergestellt sein oder dieses Material umfassen.

Erfindungsgemäß kann vorgesehen sein, dass der Bodenbereich, der Seitenwandbereich und der Schürzenbereich zumindest teilweise transparent oder durchscheinend sind.

Die Erfindung erstreckt sich weiters auf ein Verfahren zur Herstellung einer erfindungsgemäßen Kunststoffauskleidung, wobei zur Herstellung des Bodenbereichs zunächst eine erste Schicht umfassend eine Folie aus einem Fluorpolymer, insbesondere Tetrafluorethylen-Hexafluorpropylen-Copolymer (FEP) oder Polytetrafluorethylen (PTFE), mit einer zweiten Schicht aus Polyethylen durch einen medizinischen Klebstoff verklebt wird, und dieser Verbund in einem zweiten Schritt thermisch vollflächig mit einer dritten Schicht verschweißt wird. Bei der dritten Schicht kann es sich vorzugsweise um den Seitenwandbereich handeln, sodass der Bodenbereich durch den Schweißvorgang vollflächig mit dem Seitenwandbereich verbunden wird. Bei der dritten Schicht kann es sich aber auch um eine andere Schicht handeln, die mit dem Seitenwandbereich verbunden ist oder in einem weiteren Verfahrensschritt verbunden wird. In einem weiteren Verfahrensschritt wird der Seitenwandbereich mit dem Schürzenbereich verbunden, vorzugsweise durch eine umlaufende Schweißnaht verschweißt.

Erfindungsgemäß kann vorgesehen sein, dass die zur Herstellung des Bodenbereichs verwendete erste Schicht eine Dicke von 20 µm bis 40 µm, vorzugsweise 30 µm, und die zweite Schicht eine Dicke von 60 µm bis 100 µm, vorzugsweise 80 µm, aufweist.

Weitere erfindungsgemäße Merkmale ergeben sich aus den Patentansprüchen, der Beschreibung der Ausführungsbeispiele und den Figuren. Im Folgenden wird die Erfindung an Hand eines nicht ausschließlichen Ausführungsbeispiels näher erläutert.

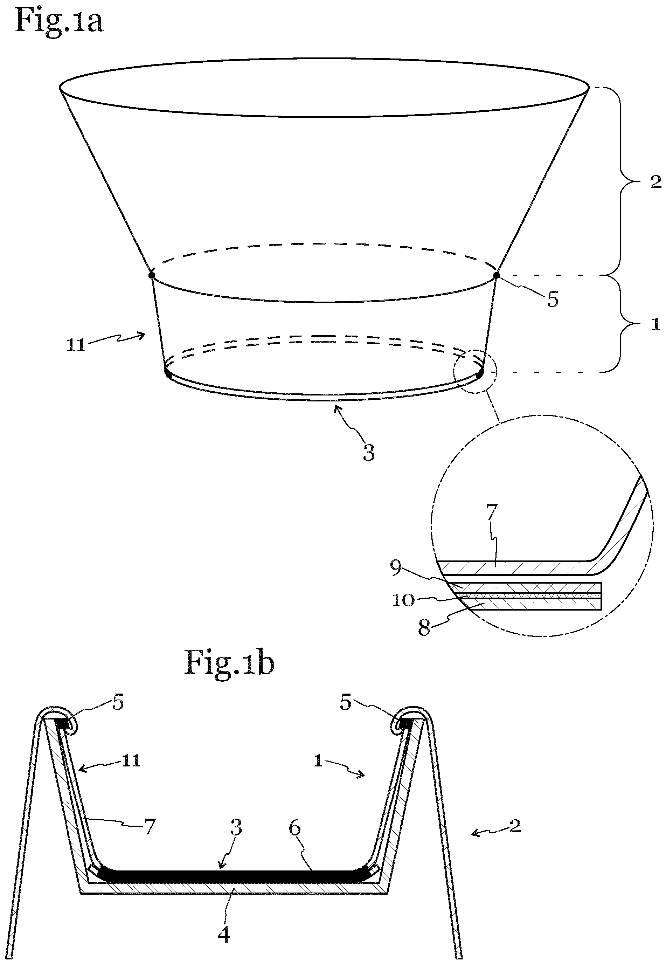

Fig. 1a zeigt eine Ausführungsform einer erfindungsgemäßen Kunststoffauskleidung. Die Kunststoffauskleidung ist im Wesentlichen sackförmig und umfasst einen Schalenbereich 1 sowie einen sich konisch erweiternden Schürzenbereich 2, die entlang einer umlaufenden thermischen Schweißnaht 5 miteinander verbunden sind. Bei der Verwendung der Kunststoffauskleidung wird der Schalenbereich 1 in eine Schale 4 eingebracht, und der Schürzenbereich um die obere Öffnung der Schale 4 geklappt, sodass der Schürzenbereich den Unterbau der Schale abdeckt. Zu diesem Zweck ist der Schalenbereich 1 der Form der Schale 4 angepasst oder anpassbar, und der Schürzenbereich 2 weist einen sich erweiternden Umfang auf, um das Umklappen zu erleichtern. Der Schürzenbereich 2 wird durch eine herkömmliche transparente Folie aus Polyethylen mit einer Dicke von etwa 60 µm gebildet.

Der Schalenbereich 1 unterteilt sich in einen im Wesentlichen ebenen Bodenbereich 3 zum direkten Kontakt mit dem beheizten Boden der Schale 4 und einen, im vorliegenden Ausführungsbeispiel sich konisch erweiternden Seitenwandbereich 11 zur Abdeckung der Seitenwand der Schale 4.

Der Seitenwandbereich 11 ist als Folie ausgebildet, die zumindest Polyamid und Polyethylen umfasst. Der Seitenwandbereich 11 weist im vorliegenden Ausführungsbeispiel eine Dicke von etwa 150 µm auf. In einem bevorzugten Ausführungsbeispiel der Erfindung ist der Seitenwandbereich 11 aus dem Material NICE XX8 des Unternehmens WIPAK gefertigt. Das Material NICE XX8 kann insbesondere einen Verbund aus Polyamid mit einer beidseitigen Versiegelung aus Polyethylen umfassen.

Der Bodenbereich 3 ist als flexible und transparente oder zumindest durchscheinende Folie ausgebildet und umfasst ein thermisch beständiges Fluorpolymer. Das Fluorpolymer befindet sich bevorzugt auf der äußeren Seite des Bodenbereichs 3, das ist jene Seite, die nach dem Einlegen in die Schale 4 in direkten Kontakt mit dem Boden der Schale 4 tritt. Dadurch wird die hohe Temperaturbeständigkeit des Materials Fluorpolymer gegenüber dem heißen Schalenboden ausgenutzt, sodass diese Seite des Bodenbereichs 3 als Wärmeschutzschicht dient.

In einem Ausführungsbeispiel kann das Fluorpolymer als Tetrafluorethylen-Hexafluorpropylen-Copolymer (FEP) ausgeführt sein. In einem anderen Ausführungsbeispiel kann das Fluorpolymer als Polytetrafluorethylen (PTFE, Teflon) ausgeführt sein.

In Fig. 1a ist ein Detail aus dem erfindungsgemäßen Herstellungsverfahren der erfindungsgemäßen Kunststoffauskleidung dargestellt. Bei der Herstellung der Kunststoffauskleidung wird zur Herstellung des Bodenbereichs 3 eine erste Schicht 8 umfassend eine Folie aus Fluorpolymer, insbesondere Tetrafluorethylen-Hexafluorpropylen-Copolymer (FEP) oder Polytetrafluorethylen (PTFE), mit einer zweiten Schicht 9 aus Polyethylen durch einen medizinischen Klebstoff 10 vollflächig verklebt.

Dieser Verbund wird in einem zweiten Schritt thermisch vollflächig mit einer dritten Schicht 7 verschweißt. Im dargestellten Ausführungsbeispiel ist die dritte Schicht 7 identisch mit der Folie im Seitenwandbereich 11. In anderen Ausführungsbeispielen kann es sich bei der dritten Schicht 7 um eine separate, mit dem Seitenwandbereich 11 verbundene Folie handeln.

Diese thermische Verschweißung der einzelnen Schichten im Bodenbereich 3 führt dazu, dass der Bodenbereich 3 der erfindungsgemäßen Kunststoffauskleidung eine einzige Schicht bildet, die sowohl die erforderliche mechanische Festigkeit, als auch in jenem Bereich, der mit der Schale in Kontakt tritt, die erforderliche hohe Temperaturbeständigkeit aufweist. Dabei ist der resultierende Bodenbereich 3 dünn genug, um eine ausreichende Wärmeleitfähigkeit zu gewährleisten.

Im vorliegenden Ausführungsbeispiel weist die erste Schicht 8 eine Dicke von etwa 30 µm, die zweite Schicht 9 eine Dicke von etwa 80 µm, und die dritte Schicht 7 eine Dicke von etwa 150 µm auf, sodass die Gesamtdicke der resultierenden Folie im Bodenbereich 3 etwa 260 µm beträgt. Dies ist einerseits dick genug, um ausreichend Stabilität gegen mechanische Beanspruchung zu gewährleisten, und andererseits dünn genug, um ausreichend Wärme von der beheizten Schale 4 an die zu wärmende Flüssigkeit zu leiten.

Fig. 1b zeigt einen Schnitt durch eine erfindungsgemäße Kunststoffauskleidung, die in eine Schale 4 eingelegt ist. Im Bodenbereich 3 ist angedeutet, dass die oben angeführten Schichten miteinander vollflächig thermisch verschweißt sind, sodass der Bodenbereich einlagig ist und somit einen nahtlosen Übergang vom Boden der Schale 4 zur zu erwärmenden Flüssigkeit gewährleistet. Der Seitenwandbereich 11 passt sich der Form der Seitenwand der Schale 4 an. Zu diesem Zweck ist der Seitenwandbereich 11 flexibel und leicht konisch nach außen erweitert. Der Schürzenbereich 2 ist über eine umlaufende Schweißnaht 5 mit dem Seitenwandbereich 11 verbunden und ist im dargestellten Zustand über den oberen Rand der Schale 4 nach unten geklappt, um den Unterbau der Schale zu bedecken. Bodenbereich 3, Seitenwandbereich 11 und Schürzenbereich 2 sind transparent oder zumindest durchscheinend, um dem Benutzer die Kontrolle der Auskleidung und den Zustand der darunterliegenden Schale 4 zu ermöglichen.

Die Erfindung beschränkt sich nicht auf den dargestellten Verwendungszweck der Kunststoffauskleidung im medizinischen Bereich sondern umfasst auch die Verwendung erfindungsgemäßer Kunststoffauskleidungen für andere Einsatzzwecke.

## Patentansprüche

1. Kunststoffauskleidung für eine beheizbare Schale (4), umfassend einen der Form der Schale (4) anpassbaren Schalenbereich (1) und einen an den Schalenbereich (1) anschließenden Schürzenbereich (2) zur Abdeckung eines Unterbaus der Schale (4), wobei der Schalenbereich (1) einen Seitenwandbereich (11) zur Abdeckung der Seitenwand der Schale (4) und einen Bodenbereich (3) zur Abdeckung des Bodens der Schale (4) umfasst, **dadurch gekennzeichnet, dass**
- der Schürzenbereich (2) Polyethylen umfasst, und
- der Seitenwandbereich (11) zumindest Polyamid und Polyethylen umfasst,
- und der Bodenbereich (3) zumindest ein Fluorpolymer, insbesondere Tetrafluorethylen-Hexafluorpropylen-Copolymer (FEP) oder Polytetrafluorethylen (PTFE), umfasst.
- und dass der Seitenwandbereich (11) und der Bodenbereich (3) als flexible, transparente Folien ausgebildet sind.

2. Kunststoffauskleidung nach Anspruch 1, **dadurch gekennzeichnet, dass** der Seitenwandbereich (11), der Bodenbereich (3) und der Schürzenbereich (2) einlagig sind.

3. Kunststoffauskleidung nach Anspruch 1 oder 2, **dadurch gekennzeichnet, dass** der Bodenbereich (3) eine höhere Dicke als der Seitenwandbereich (11), und der Seitenwandbereich (11) eine höhere Dicke als der Schürzenbereich (2) aufweist.

4. Kunststoffauskleidung nach einem der Ansprüche 1 bis 3, **dadurch gekennzeichnet, dass** der Schürzenbereich (2) eine Dicke im Bereich von 40µm bis 80 µm, vorzugsweise 60 µm aufweist.

5. Kunststoffauskleidung nach einem der Ansprüche 1 bis 4, **dadurch gekennzeichnet, dass** der Seitenwandbereich (11) eine Dicke im Bereich von 120 µm bis 180 µm, vorzugsweise 150 µm, aufweist.

6. Kunststoffauskleidung nach einem der Ansprüche 1 bis 5, **dadurch gekennzeichnet, dass** der Bodenbereich (3) eine Dicke im Bereich von 200 µm bis 300 µm, vorzugsweise 260 µm, aufweist.

7. Kunststoffauskleidung nach einem der Ansprüche 1 bis 6, **dadurch gekennzeichnet, dass** der Schalenbereich (1) an den Schürzenbereich (2) angeformt, insbesondere über eine thermische Schweißnaht (5) verbunden ist.

8. Kunststoffauskleidung nach einem der Ansprüche 1 bis 7, **dadurch gekennzeichnet, dass** der Seitenwandbereich (11) einen Verbund aus Polyamid mit einer beidseitigen Versiegelung aus Polyethylen umfasst.

9. Kunststoffauskleidung nach einem der Ansprüche 1 bis 8, **dadurch gekennzeichnet, dass** der Bodenbereich (3), der Seitenwandbereich (11) und der Schürzenbereich (2) zumindest teilweise transparent ist.

10. Verfahren zur Herstellung einer Kunststoffauskleidung nach einem der Ansprüche 1 bis 9, **dadurch gekennzeichnet, dass** zur Herstellung des Bodenbereichs (3) eine erste Schicht (8) umfassend eine Folie aus einem Fluorpolymer, insbesondere Tetrafluorethylen-Hexafluorpropylen-Copolymer (FEP) oder Polytetrafluorethylen (PTFE), mit einer zweiten Schicht (9) aus Polyethylen durch einen medizinischen Klebstoff (10) verklebt wird, und dieser Verbund thermisch vollflächig mit einer dritten Schicht (7) verschweißt wird , wobei die dritte Schicht (7) einstückig mit der Folie im Seitenwandbereich (11) ist oder diese umfasst, und wobei der Seitenwandbereich (11) und der Bodenbereich (3) als flexible transparente Folie ausgebildet sind.

11. Verfahren zur Herstellung einer Kunststoffauskleidung nach Anspruch 10, **dadurch gekennzeichnet, dass** die erste Schicht (8) eine Dicke von 20 µm bis 40 µm, vorzugsweise 30 µm, und die zweite Schicht (9) eine Dicke von 60 µm bis 100 µm, vorzugsweise 80 µm, aufweist.

## Claims

1. A plastic liner for a heatable basin (4) comprising a basin portion (1) that can be adapted to the shape of the basin (4) and a drape portion (2) attached to the basin portion (1) for covering a base structure of the basin (4), wherein the basin portion (1) comprises a side wall region (11) for covering the side wall of the basin (4) and a bottom region (3) for covering the bottom of the basin (4),
**characterized in that**
- the drape portion (2) comprises polyethylene and
- the side wall region (11) at least comprises polyamide and polyethylene, and
- the bottom region (3) comprises at least one fluoropolymer, in particular tetrafluoroethylene hexafluoropropylene copolymer (FEP) or polytetrafluoroethylene (PTFE),
- and that the side wall region (11) and the bottom region (3) are configured as flexible, transparent films.

2. The plastic liner according to claim 1, **characterized in that** the side wall region (11), the bottom region (3) and the drape portion (2) are one-ply.

3. The plastic liner according to claim 1 or 2, **characterized in that** the bottom region (3) has a greater thickness than the side wall region (11) and the side wall region (11) has a greater thickness than the drape portion (2).

4. The plastic liner according to one of claims 1 to 3, **characterized in that** the drape portion (2) has a thickness in the range of 40 µm to 80 µm, preferably 60 µm.

5. The plastic liner according to one of claims 1 to 4, **characterized in that** the side wall region (11) has a thickness in the range of 120 µm to 180 µm, preferably 150 µm.

6. The plastic liner according to one of claims 1 to 5, **characterized in that** the bottom region (3) has a thickness in the range of 200 µm to 300 µm, preferably 260 µm.

7. The plastic liner according to one of claims 1 to 6, **characterized in that** the basin region (1) is moulded on the drape portion (2), in particular is connected via a thermal weld seam (5).

8. The plastic liner according to one of claims 1 to 7, **characterized in that** the side wall region (1) comprises a composite of polyamide with a two-sided polyethylene seal.

9. The plastic liner according to one of claims 1 to 8, **characterized in that** the bottom region (3), the side wall region (11) and the drape portion (2) is at least partially transparent.

10. A method of producing a plastic liner according to one of claims 1 to 9, **characterized in that** in order to produce the bottom region (3) a first layer (8) comprising a film made of a fluoropolymer, in particular tetrafluoroethylene hexafluoropropylene copolymer (FEP) or polytetrafluoroethylene (PTFE), is bonded to a second layer (9) made of polyethylene by a medical adhesive (10) and this composite is welded to a third layer (7) in a thermally full-surface manner, wherein the third layer (7) is integral with the film in the side wall region (11) or encloses said film, and wherein the side wall region (11) and the bottom region (3) are configured as a flexible transparent film.

11. The method of producing a plastic liner according to claim 10, **characterized in that** the first layer (8) has a thickness of 20 µm to 40 µm, preferably 30 µm, and the second layer (9) has a thickness of 60 µm to 100 µm, preferably 80 µm.

## Revendications

1. Habillage en matière plastique pour une cuvette chauffable (4), comprenant une zone de cuvette (1) adaptable à la forme de la cuvette (4) et une zone de tablier (2) se raccordant à la zone de cuvette (1) pour recouvrir une structure inférieure de cuvette (4), la zone de cuvette (1) comprenant une zone de paroi latérale (11) pour recouvrir la paroi latérale de la cuvette (4) et une zone de fond (3) pour recouvrir le fond de la cuvette (4), **caractérisé en ce que**
- la zone de tablier (2) comprend du polyéthylène, et
- la zone de paroi latérale (11) comprend au moins du polyamide et du polyéthylène, et la zone de fond (3) comprend au moins un polymère fluoro-carboné, notamment un copolymère de tétrafluoréthylène-hexafluoropropylène (FEP) ou du polytétrafluoréthylène (PTFE),
- et **en ce que** la zone de paroi latérale (11) et la zone de fond (3) sont constituées en tant que feuilles souples, transparentes.

2. Habillage en matière plastique selon la revendication 1, **caractérisé en ce que** la zone de paroi latérale (11), la zone de fond (3) et la zone de tablier (2) sont en une seule couche.

3. Habillage en matière plastique selon la revendication 1 ou 2, **caractérisé en ce que** la zone de fond (3) comporte une épaisseur plus élevée que la zone de paroi latérale (11) et la zone de paroi latérale (11) une épaisseur plus élevée que la zone de tablier (2).

4. Habillage en matière plastique selon l'une quelconque des revendications 1 à 3, **caractérisé en ce que** la zone de tablier (2) comporte une épaisseur se situant dans une plage de 40µm à 80 µm, de préférence 60pm.

5. Habillage en matière plastique selon l'une quelconque des revendications 1 à 4, **caractérisé en ce que** la zone de paroi latérale (11) comporte une épaisseur se situant dans une plage de 120 µm à 180 µm, de préférence 150 µm.

6. Habillage en matière plastique selon l'une quelconque des revendications 1 à 5 **caractérisé en ce que** la zone de fond (3) comporte une épaisseur se situant dans une plage de 200 µm à 300 µm, de préférence 260 µm.

7. Habillage en matière plastique selon l'une quelconque des revendications 1 à 6, **caractérisé en ce que** la zone de cuvette (1) est conformée sur la zone de tablier (2), est notamment reliée par le biais d'un cordon de soudure thermique (5).

8. Habillage en matière plastique selon l'une quelconque des revendications 1 à 7, **caractérisé en ce que** la zone de paroi latérale (11) comprend un composite de polyamide avec un scellement étanche bilatérale en polyéthylène.

9. Habillage en matière plastique selon l'une quelconque des revendications 1 à 8, **caractérisé en ce que** la zone de fond (3), la zone de paroi latérale (11) et la zone de tablier (2) sont au moins en partie transparentes.

10. Procédé pour la fabrication d'un habillage en matière plastique selon l'une quelconque des revendications 1 à 9, **caractérisé en ce que** pour la fabrication de la zone de fond (3), une première couche (8) comprenant une feuille d'un polymère fluoro-carboné, notamment un copolymère de tétrafluoréthylène-hexafluoropropylène (FEP) ou du polytétrafluoréthylène (PTFE) est collée à une deuxième couche (9) de polyéthylène par une colle médicale (10) et ce composite est soudé thermiquement sur toute la surface à une troisième couche (7), la troisième couche (7) formant une seule pièce avec la feuille dans la zone de la paroi latérale (11) ou comprenant celle-ci et la zone de paroi latérale (11) et la zone de fond (3) étant constituées en tant que feuille souple transparente.

11. Procédé pour la fabrication d'un habillage en matière plastique selon la revendication 10, **caractérisé en ce que** la première couche (8) comporte une épaisseur de 20 µm à 40 µm, de préférence 30 µm et la deuxième couche (9) une épaisseur de 60 µm à 100 µm, de préférence 80 µm.
